# EUROPÄISCHE PATENTANMELDUNG

(11) **EP 3 984 480 A1**
(43) Veröffentlichungstag der Anmeldung: **20.04.2022**
(21) Anmeldenummer: 20202362.8
(22) Anmeldetag: 16.10.2020
(51) Int. Cl.: A61B 18/04, A61B 18/14

(54) **MULTILUMENSONDE**

(71) Anmelder: Erbe Elektromedizin GmbH, 72072 Tübingen (DE)
(72) Erfinder: HEYM, Johannes, 72070 Tuebingen (DE); WALZ, Martin, 72076 Tuebingen (DE)
(74) Vertreter: Rüger Abel Patentanwälte PartGmbB

(57) **Zusammenfassung**

Ein Instrument (10) weist einen Sondenschlauch (14) auf, in dessen Zentrum ein Leiter (31) zur elektrischen Versorgung einer Elektrode (30) vorgesehen ist. Konzentrisch um den Leiter (31) herum, sind mehrere gasführende Lumen (22, 23, 24) angeordnet, die durch Trennwände (18, 19, 20) voneinander isoliert sind. Die Trennwände (18, 19, 20) halten einen zentralen den Leiter (31) beherbergenden Mittelabschnitt (21) der maßgeblich zur elektrischen Isolation des Leiters (31) dient.

Mit diesem Sondendesign lassen sich besonders flexible und besonders schlanke Sonden schaffen.

## Beschreibung

Die Erfindung betrifft ein Instrument zur Behandlung von biologischem Gewebe insbesondere zur Argon-Plasma-koagulation desselben im endoskopischen Einsatz.

Endoskopisch verwendbare Instrumente zur Argonplasmakoagulation sind prinzipiell bekannt. Die WO 2008/090004 A1 offenbart ein solches Instrument mit einem flexiblen Schlauch und zwei darin vorgesehenen Elektroden, zwischen denen ein Lichtbogen gezündet wird. Das schlauchartige Instrument weist ein oder auch zwei Lumen auf, wobei bei der zweilumigen Variante ein äußerer Sondenschlauch und zwei innere Schläuche vorhanden sind, in denen die beiden Lumen ausgebildet sind. Jedem Lumen ist eine der beiden Elektroden zugeordnet. Die jeweilige Elektrode ist mit einem Leiter verbunden, der sich dann ohne eigene Isolation über die gesamte Länge des Instruments durch das ihm zugeordnete Lumen erstreckt. Die Elektrode ist mittig in einer Gasauslassöffnung des jeweiligen Lumens gehalten. Der äußere Sondenschlauch weist dabei einen Ovalquerschnitt auf.

Aus der WO 2006/119892 A1 ist ein mehrlumiges Instrument bekannt, bei dem in einem Sondenschlauch 11 konzentrisch ein weiterer Schlauch angeordnet ist, in dem eine Elektrode gehalten ist. Zur Lagerung derselben in dem inneren Schlauch weist diese einen schraubenförmig gewundenen Abschnitt auf, mit dem sie sich an der Innenwand des inneren Schlauchs abstützt. Zur Fixierung des inneren Schlauchs in der Mitte des äußeren Schlauchs sind radial orientierte Abstandshalter vorgesehen.

Ebenfalls ein bipolares Instrument ist aus der EP 3 205 301 B1 bekannt. Dieses Instrument umfasst einen Sondenschlauch mit einem Lumen und zwei in den Sondenschlauch eingebetteten Elektroden. Während eine der Elektrode mit einem auf einer Keramikhülse sitzenden Metallring versehen ist, steht die andere Elektrode mittig in dem zentralen Durchgang der Keramikhülse. Die keramische Hülse bildet einen elektrischen Isolator, so dass sich im Innenraum derselben eine elektrische Barriereentladung und somit ein nicht thermisches Plasma bildet.

Aus der EP 0 353 177 A1 ist ein für den offenchirurgischen Einsatz vorgesehenes Handinstrument bekannt, das an seinem distalen Ende einen Austrittskanal und eine darin angeordnete Elektrode aufweist. Die zur Zuführung von Gas an dieses Handinstrument vorgesehene Leitung, weist mehrere Lumen auf.

Weiterer Stand der Technik wird von der EP 0 738 519 A1**, der** JP 2002-301088 A und der EP 3 412 234 A1 gebildet.

Sonden zur Erzeugung von thermischem Plasma unterliegen einer erheblichen Wärmebeanspruchung, was der Materialauswahl bei der Gestaltung solcher Sonden Grenzen setzt. Außerdem sind zur Plasmaerzeugung hohe elektrische Spannungen erforderlich die zur Erreichung der nötigen Durchschlagfestigkeit eine große Wandstärke des Sondenschlauchs erfordern. Dies ist bei der geometrischen Gestaltung der Sonden zu berücksichtigen und führt aufgrund der üblichen Designs typischerweise zu einer störenden Steifigkeit der Sonde.

Es ist deswegen Aufgabe der Erfindung, ein Grundkonzept für ein Instrument anzugeben, das erweiterte Gestaltungsmöglichkeiten erbringt.

### Diese Aufgabe wird mit dem Instrument nach Anspruch 1 gelöst:

Das erfindungsgemäße Instrument ist insbesondere als monopolares Instrument ausbildbar, das zur Plasmakoagulation, insbesondere zur Argon-Plasma-Koagulation von biologischem Gewebe geeignet ist. Das Instrument ist insbesondere eine flexible Sonde. In dem sich am distalen Ende derselben ausbildenden oder aus diesem ausströmenden Plasma fließt bei dem erfindungsgemäßen Instrument ein Strom zwischen der (vorzugsweise einzigen) Elektrode des Instruments und dem biologischem Gewebe.

Das Instrument (die Sonde) umfasst einen Sondenschlauch, der mindestens zwei, vorzugsweise drei oder mehrere an eine Gasversorgungseinrichtung anschließbare Lumen aufweist. Diese Lumen erstrecken sich vorzugsweise durch den gesamten Sondenschlauch hindurch von dem proximalen Ende bis zu am distalen Ende gelegenen Gasaustrittsöffnungen. Vorzugsweise ist in keinem der Lumen ein stromführendes Element oder ein elektrischer Leiter oder eine Elektrode angeordnet.

An dem distalen Ende des Sondenschlauchs ist vorzugsweise zentrisch eine Elektrode angeordnet, deren aktives Ende in diesem Bereich ohne elektrische Isolation frei liegt. Das aktive Ende der Elektrode ist derjenige Bereich derselben, der mit dem aus den Gasaustrittsöffnungen austretenden Gasstrom in Berührung steht und diesen Gasstrom ionisiert. Das aktive Ende der Elektrode kann dabei erhebliche Temperaturen von bis zu mehreren 100°C erreichen. Die Gasaustrittsöffnungen sind um die Elektrode herum gruppiert.

Der Sondenschlauch weist dank seines mehrlumigen Aufbaus typischerweise ein äußeren hohlzylindrischen Abschnitt, einen konzentrisch darin angeordneten, vorzugsweise etwa zylindrischen Naben- oder Mittelabschnitt und dazwischen nach Art von Speichen angeordnete vorzugsweise flache Trennwände auf. Vorzugsweise weist jede Trennwand vom Mittelabschnitt bis zum Außenabschnitt eine im Wesentlichen konstante Dicke auf. Vorzugsweise variiert die Dicke um weniger als 20%. Der Mittelabschnitt, die Trennwände und der äußere hohlzylindrische Abschnitt sind vorzugsweise Teile ein und desselben Kunststoffschlauchs, die aus gleichem Material bestehen und nahtlos ineinander übergehen. Es ergeben sich ein hohes elektrisches Isolationsvermögen eine hohe Flexibilität. Solche Schläuche gestatten die Einnahme enger Biegeradien.

Die elektrische Isolation wird ausgehend von dem Leiter in Radialrichtung nach außen zunächst und hauptsächlich durch den Mittelabschnitt erbracht. Der Radius des Mittelabschnitts ist vorzugsweise gleich wie oder größer als die äußere Wandstärke des Schlauchs.

Die Maximierung des Durchmessers des Mittelabschnitts beeinträchtigt die Flexibilität des Sondenschlauchs kaum, denn der Mittelabschnitt trägt wenig zur Biegesteifigkeit der Sonde bei. Hingegen kann der äußere hohlzylindrische Mantel relativ dünnwandig ausgebildet werden. Damit können trotz hohen, durch den Mittelabschnitt erbrachten Isolationsvermögens Fluidkanäle erzeugt werden, die einen großen freien Strömungsquerschnitt aufweisen.

Der Sondenschlauch kann aus einem Kunststoff hergestellt werden, der elektrisch weniger durchschlagfest ist und/oder der einen höheren Elastizitätsmodul aufweist, als der sonst für Argon-Plasma- Sonden eingesetzte Werkstoff, wie z.B. Fluorkunststoffe, insbesondere PTFE und FEP.

Es ist möglich, den Mittelabschnitt an seiner Außenseite und/oder den Mantelabschnitt an seiner Innenseite mit einer Metallisierung oder Metall-Inlays zu versehen, um Äquipotenzialflächen zu erzeugen. Auch damit lässt sich die Spannungsdurchschlagfestigkeit des Sondenschlauchs weiter erhöhen.

Jedenfalls wird es bevorzugt, die radiale Dicke des Mittelabschnitts größer zu bemessen, als die radiale Dicke des Mantelabschnitts, so dass die elektrische Isolation vorwiegend von dem Mittelabschnitt erbracht wird.

Die Gasaustrittsöffnungen sind vorzugsweise konzentrisch um die Elektrode herum angeordnet. Die zwischen den Lumen des Sondenschlauchs angeordneten Trennwände können gegen die Radialrichtung geneigt sein. Vorzugsweise sind dabei alle Trennwände in der gleichen Richtung geneigt. Die Lumen können im Wesentlichen einen Querschnitt eines Dreiecks mit bogenförmigen Kanten (zwei konvexe, eine konkave) haben. Anstelle spitzer Ecken können auch Rundungen vorgesehen sein. Jede der o.g. Maßnahmen für sich trägt dazu bei, dass der Schlauch in allen Radialrichtungen etwa gleich flexibel und gleich unempfindlich gegen das Schließen eines gasführenden Lumens durch Abknicken des Sondenschlauchs ist. Auch tragen die gegen die Radialrichtung geneigten Trennwände zur Flexibilität des Sondenschlauchs bei und führen auch dazu, dass die Elektrode gleichmäßig von Gas umspült wird.

Durch eine Krümmung der Trennwände, die im Querschnitt als Krümmung um eine senkrecht auf der Querschnittsebene stehende Achse sichtbar wird, wird die Flexibilität des Sondenschlauchs begünstigt und die elektrische Isolationsfestigkeit insbesondere auch an Knickstellen des Sondenschlauchs sichergestellt. Die Trennwände legen sich beim Abwinkeln des Sondenschlauchs zwischen den Mittelabschnitt und den Mantelabschnitt und erhöhen dort die Durchschlagfeldstärke. Dies kommt der elektrischen Durchschlagfestigkeit zugute.

In den Mittelabschnitt des Sondenschlauchs kann ein ansonsten nicht weiter isolierter Leiter eingebettet sein und ihn dadurch elektrisch isolieren. Anstelle eines nicht isolierten Leiters kann jedoch auch ein isolierter Leiter in den Mittelabschnitt eingebettet sein, so dass dieser elektrische Leiter von einer mehrschichtigen Isolation, bestehend aus unterschiedlichen Materialien umgeben ist. Auch dies kann zur Verbesserung der elektrischen Isolation, oder umgekehrt zur Miniaturisierung des Sondendesigns genutzt werden. Der Leiter kann ein Draht oder eine Litze aus Metall oder einem elektrisch leitfähigen Kunststoff sein. Der mehrschichtige Aufbau der Isolation ist ein Konzept, dank dessen die Vielfalt der verwendbaren Werkstoffe für den Sondenschlauch vergrößert wird. Z.B. kann der Mittelabschnitt aus einem hinsichtlich seiner elektrischen Isolationsfähigkeit optimierten Werkstoff bestehen, wohingegen der Mantelabschnitt (und/oder auch die Trennwände) aus einem hinsichtlich seiner Flexibilität optimierten Werkstoff besteht.

Der Sondenschlauch weist vorzugsweise über seine gesamte Länge ausgehend von dem proximalen Ende bis zu den Gasaustrittsöffnungen einen konstanten Querschnitt auf. Die gasführenden Lumen können gerade, parallel zur Mittelachse angeordnet sein oder auch einen schraubenförmigen Verlauf haben.

Der Mantelabschnitt kann sich über die Gasaustrittsöffnungen hinaus in distaler Richtung erstrecken, so dass am distalen Ende des Instruments eine Plasmakammer gebildet ist, innerhalb derer das distale Ende der Elektrode positioniert ist. Der Mantelabschnitt kann aus dem Material des Sondenschlauchs bestehen. Es ist jedoch auch möglich, den die Plasmakammer umgebenden Endabschnitt aus einem anderen Material, beispielsweise Keramik, auszubilden.

Es ist weiter möglich, die Elektrode aus dem Sondenschlauch herausragen zu lassen und an ihrem freien distalen Ende einen Schutzkörper vorzusehen. Der Schutzkörper ist vorzugsweise ein elektrisch isolierender Körper, z.B. ein Keramikkörper, beispielsweise eine Keramikkugel oder ein sonstiger Körper. Vorzugsweise weist dieser Schutzkörper ein Durchmesser auf, der deutlich größer ist als der Elektrodendurchmesser und beispielsweise etwa mit dem Außendurchmesser des Sondenschlauchs übereinstimmt. Vorzugsweise ist der Schutzkörper an seiner distalen Endfläche gerundet und frei von Spitzen oder scharfen Kanten. Dieses Konzept eignet sich insbesondere für Radialsonden, die einen Plasmastrom in beliebiger Radialrichtung abgeben können. Bei asymmetrischer Gestaltung des Schutzkörpers, beispielsweise als schräg gestellte Keramikscheibe oder dergleichen, können auch Radialrichtungen zur bevorzugten Plasmaabgabe festgelegt werden.

Die Elektrode kann an ihrem Endabschnitt als blankes Drahtende ausgebildet sein. Beispielsweise kann der Draht aus einem Chromnickelstahl bestehen, der schlecht wärmeleitfähig ist und somit wenig Wärme in den Mittelabschnitt des Sondenschlauchs einträgt, wo er in direktem Kontakt mit dem Kunststoff des Sondenschlauchs steht. Es ist auch möglich, die sich als Draht durch den Sondenschlauch erstreckende Elektrode auf ganzer Länge oder wenigstens im Bereich ihres distalen Endes, zum Beispiel im aktiven Endabschnitt, mit einer Beschichtung zu versehen. Diese Beschichtung ist vorzugsweise aus einem elektrisch leitfähigen Material ausgebildet. Vorzugsweise handelt es sich bei dem Material um ein Metall, dessen Schmelztemperatur niedriger ist als die Schmelztemperatur der Elektrode. Beispielsweise kann die Beschichtung aus Silber oder einer Silberlegierung sein. Weiter vorzugsweise kann zwischen dem Grundmaterial der Elektrode (zum Beispiel Chromnickelstahl) und der niedrig schmelzenden Beschichtung eine weitere Schicht, zum Beispiel eine Haftvermittlungsschicht, zum Beispiel in Gestalt einer Goldschicht vorgesehen sein. Derartige Elektroden erweisen sich als standfest und sie tragen wenig Wärme in den Sondenschlauch ein. Durch die Beschichtung und die dadurch erreichte niedrigere thermische Belastung ist es nun möglich, eine Elektrode direkt im Sondenschlauch zu befestigen. Bisher z.B. verwendete Plättchenelektroden oder Nadelelektroden mit schraubenförmiger Basis haben eine erhöhte Kühlung durch Konvektion, sowie eine gewisse Beabstandung der Entladungszone von dem Schlauch bewirkt. Auf beides kann in der vorliegenden Erfindung verzichtet werden, wodurch die Gestaltung einer flexibleren und miniaturisierte Sonde möglich wird.

Weiter ist es möglich, an dem distalen Ende des sich durch den Sondenschlauch erstreckenden Drahts einen Elektrodenaufsatz vorzusehen. Dieser kann zum Beispiel mit der oben genannten Beschichtung versehen sein.

Weiterer Einzelheiten vorteilhafter Ausführungsformen der Erfindung ergeben sich aus Unteransprüchen, den Figuren der Zeichnung, sowie der zugehörigen Beschreibung. Es zeigen:
Figur 1 ein erfindungsgemäßes Instrument, angeschlossen an ein versorgende Gerät in schematisierter Perspektivdarstellung,
Figur 2 das distale Ende des Instrument in perspektivischer Veranschaulichung,
Figur 3 das Instrument nach Figur 1 und 2 in Stirnansicht,
Figur 4 das Instrument nach Figur 3 im Längsschnitt in ausschnittsweiser Darstellung,
Figur 5 eine abgewandelte Ausführungsform des Instruments nach Figur 4 im ausschnittsweisen Längsschnitt,
Figur 6 eine weiter abgewandelte Ausführungsform des Instruments nach Figur 4 in längsgeschnittener Darstellung des distalen Endes desselben,
Figur 7 das distale Ende einer weiteren Ausführungsform eines erfindungsgemäßen Instruments in teilweise geschnittener Seitenansicht,
Figur 8 und 9 Instrument mit abgewandeltem Sondenschlauch jeweils in Stirnansicht.

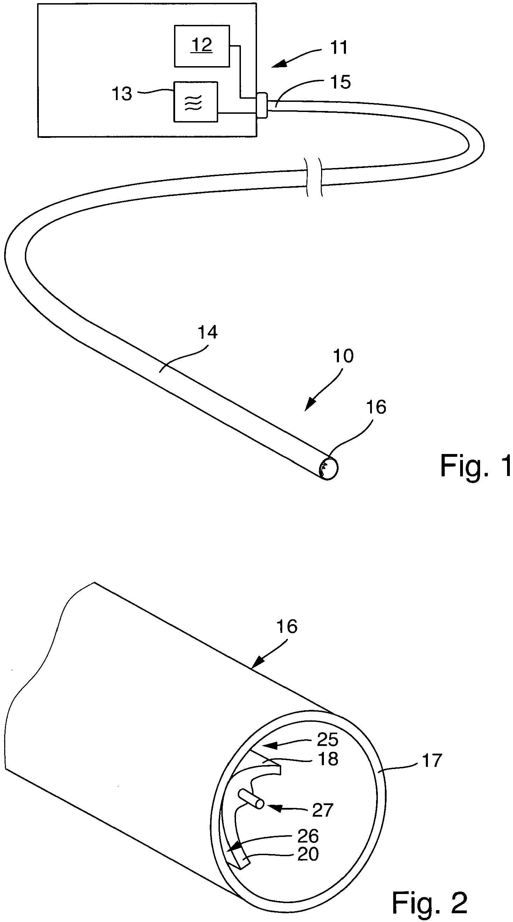

In Figur 1 sind ein chirurgisches Instrument 10 in Gestalt einer Multilumensonde veranschaulicht, die an ein speisendes Gerät 11 angeschlossen ist. Die Multilumensonde kann zur chirurgischen Behandlung eines Patienten dienen und dazu durch einen Arbeitskanal eines Endoskops in den Patienten eingeführt werden. Das Gerät 10 dient der Speisung des Instruments 10 mit dem zum Betrieb erforderlichen Medium und mit elektrischem Strom. Beispielsweise kann das Gerät 11 dazu eine Gasquelle 12 und einen elektrischen Generator 13 aufweisen. Die Gasquelle 12 kann beispielsweise eine Argonquelle sein, die durch einen in einem Druckbehälter gehaltenen Gasvorrat, beispielsweise Argonvorrat, und entsprechende Steuerelemente, wie Ventile, Druckregler und dergleichen gebildet ist. Der elektrische Generator 13 ist vorzugsweise ein HF-Generotor zur Abgabe einer hochfrequenten Wechselspannung mit gewünschter Spitzenspannung, vorzugsweise einstellbarer Modulation und/oder einstellbarer Leistung.

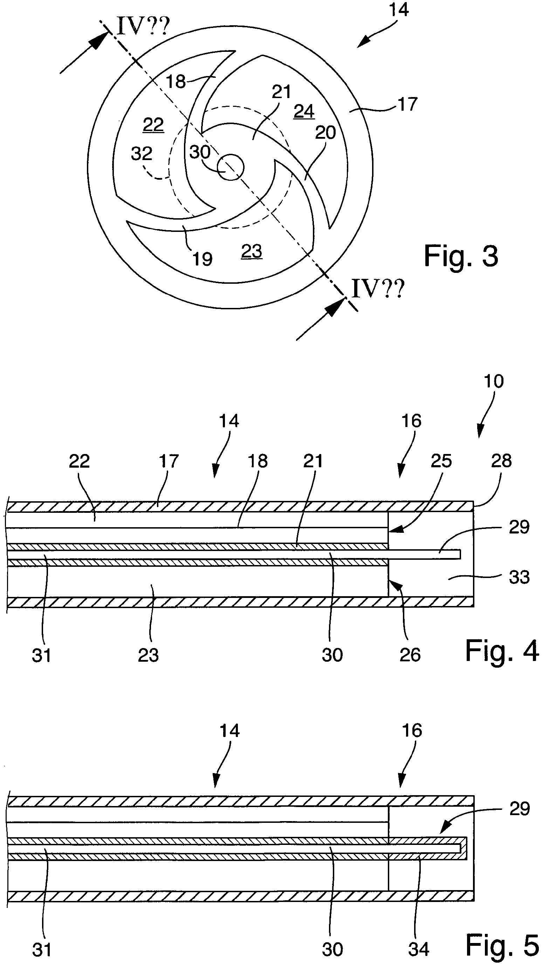

Das Instrument 10 umfasst einen Sondenschlauch 14, der sich von einem proximalen Ende 15 bis zu einem distalen Ende 16 erstreckt. Der Sondenschlauch 14 ist ein flexibler, vorzugsweise aus einem Kunststoff, beispielsweise PTFE, FEP oder auch PA, TPE, HDPE oder PP bestehender Schlauch. Der Sondenschlauch 14 weist einen außen vorzugsweise kreisförmigen Querschnitt auf, wie aus Figur 3 erkennbar ist. Alternativ kann der Querschnitt außen auch polygonal, z.B. sechseckig oder achteckig sein. Der Querschnitt wird außen von einem kreisringförmigen Mantel 17 festgelegt, von dessen Innenseite sich mehrere Trennwände 18, 19, 20 zu einem nabenartigen Mittelabschnitt 21 erstrecken, der im Zentrum des Sondenschlauchs mit vorzugsweise zylinderförmiger Außenfläche angeordnete ist. Vorzugsweise ist eine ungerade Anzahl von Trennwänden vorhanden, woraus sich eine homogene, d.h. in allen Radialrichtungen gleiche Steifigkeit ergibt. In dem Sondenschlauch 14 werden durch die Trennwände 18, 19, 20 mindestens zwei, vorzugsweise drei oder mehrere Lumen 22 bis 24 gegeneinander abgegrenzt, die sich jeweils von dem proximalen Ende 15 bis zu dem distalen Ende 16 bzw. dort vorhandenen Gasausströmöffnungen 25, 26, 27 erstrecken und um den Mittelabschnitt herum gruppieren. Je nach Werkstoff und Genauigkeit der Extrusion kann der Querschnitt der Außenfläche und/oder der Querschnitt des Mittelabschnitts aber auch polygonal ausgeprägt sein.

Wie Figur 4 erkennen lässt, sind die Gasausströmöffnungen 25, 26 (und 27) gegen das stirnseitige Ende 28 des Sondenschlauchs 14 in proximaler Richtung zurückversetzt, so dass an dem distalen Ende 16 des Sondenschlauchs 14 eine kammerartige Vertiefung gebildet ist. In diese ragt das aktive Ende 29 einer Elektrode 30, die vorzugsweise zentrisch in dem Mittelabschnitt 21 gehalten ist. Die kammerartige Vertiefung ist eine Plasmakammer, in der der Stromfluss von der Elektrode 30 auf das zu bildende Plasma übergeht.

Bei dem in Figur 4 veranschaulichten, zur Abgabe eines axialen Plasmastroms eingerichteten Instrument 10, ist das aktive Ende 29 der Elektrode 30 vollständig innerhalb des Instruments 10 und somit in der Plasmakammer angeordnet. Die Spitze des aktiven Endes 29 der Elektrode 30 ist somit proximal gegen das stirnseitige Ende 28 des Sondenschlauchs 14 zurückversetzt. Die Elektrode kann auch auf einer Ebene mit der Stirnseite des Sondenschlauchs 14 stehen.

Von der Elektrode 30 ausgehend erstreckt sich ein elektrischer Leiter vorzugsweise zentrisch durch den Mittelabschnitt 21 bis zu dem proximalen Ende 15, um dort mit einem Pol des Generators 13 verbunden zu sein. Der andere Pol des Generators 30 ist mit einer nicht weiter veranschaulichten Neutralelektrode verbindbar oder verbunden, die an dem Patienten zur Stromrückleitung anbringbar ist. Es handelt sich bei dem Instrument 10 somit um ein monopolares Instrument, bei dem der Patient Teil des Behandlungsstromkreises ist.

Die Elektrode 30 kann mit der sich in proximaler Richtung weg erstreckenden elektrischen Zuleitung 31 einteilig ausgebildet und somit Teil derselben sein. Die Elektrode 30 kann aber auch durch ein gesondertes Metallelement gebildet sein, das mit der Zuleitung 31 verbunden ist. Vorzugsweise besteht die Elektrode 30 aus einem wenig wärmeleitfähigen Material, wie beispielsweise rostfreiem Stahl, insbesondere einem Chromnickelstahl, beispielsweise mit folgender Zusammensetzung:

| | Fe | C | Cr | Mn | P | S | Si | Ni | N | Mo |
|---|---|---|---|---|---|---|---|---|---|---|
| min | | 0,05 | 16,0 | | | | | 6,0 | | |
| max | 47,605 | 0,15 | 19,0 | 2,0 | 0,045 | 0,15 | 2,0 | 9,5 | 0,11 | 0,8 |

Zumindest das aktive Ende 29 oder auch die gesamte Elektrode 30 kann mit einer Beschichtung versehen sein. Die Beschichtung kann sich auch über die gesamte Länge des Leiters 31 erstrecken. Die Beschichtung ist vorzugsweise eine Metallbeschichtung, deren Schmelztemperatur niedriger ist, als die Schmelztemperatur der Elektrode 30 bzw. des aktiven Endes 29. Insbesondere kann die Beschichtung durch eine Silberschicht gebildet sein. Zwischen der Silberschicht und dem Material der Elektrode bzw. des aktiven Endes 29 der Elektrode 30, kann eine Haftschicht vorgesehen sein. Diese Haftschicht besteht vorzugsweise aus einem Material, dessen Schmelztemperatur niedriger ist als die Schmelztemperatur der Elektrode 30 oder des aktiven Endes 29 derselben. Vorzugsweise ist die Schmelztemperatur der Haftschicht jedoch zumindest so hoch wie die Schmelztemperatur der Beschichtung. Die Haftschicht kann beispielsweise eine Goldschicht sein.

In Betrieb stehen die Elektrode 30 und der Leiter 31 unter einer hohen Spannung, die mehrere 100 V bis mehrere 1000 V betragen kann. Zur elektrischen Isolation des Leiters 31 weist der Mittelabschnitt 21 in Radialrichtung eine Dicke auf, die vorzugsweise größer ist, als die in Radialrichtung zu messende Dicke des Mantels 17. Sowohl der Mittelabschnitt 21, als auch der Mantel 17 tragen zur elektrischen Isolation des Leiters 31 gegen das umgebende Endoskop und/oder das umgebende biologische Gewebe bei. Infolge der genannten Aufteilung der Materialstärke zu Gunsten des Mittelabschnitts 21, ergibt sich eine hohe Flexibilität des Sondenschlauchs 14. Außerdem wird der Strömungsquerschnitt der Lumen 22, 23, 24 möglichst groß. Bedarfsweise kann zum Beispiel die radiale Dicke des Mittelabschnitts 21 erheblich erhöht werden, wie in Figur 3 durch einen gestrichelten Kreis 32 veranschaulicht ist. Dies verbessert die elektrische Isolierung des Leiters 31 erheblich, ohne den Strömungsquerschnitt der Lumen 22, 23, 24 wesentlich zu beeinträchtigen.

Zur weiteren Erhöhung der Flexibilität und/oder zur Vergleichmäßigung der Biegsamkeit in allen Radialrichtung und zur Vermeidung eines Lumenverschlusses beim Abwinkeln des Sondenschlauchs 14, können die Trennwände 18, 19, 20, wie aus Figur 3 ersichtlich, zur Radialrichtung geneigt und auch gekrümmt ausgebildet sein. Wird ein solcher Sondenschlauch 14 mit geringem Biegeradius gebogen, können sich die Trennwände 18, 19, 20 jeweils an einer Seite der Biegung an den Mittelabschnitt 21 anlegen, wohingegen sich die anderen Trennwände 19, 20 aufrichten können. Dabei sind immer mindestens ein, meistens zwei oder drei der Lumen offen, so dass der Gasstrom darin frei in distaler Richtung fließen kann. Ein Abknicken des Sondenschlauchs 14 mit Blockade der Lumen findet nicht statt.

Das insoweit beschriebene Instrument 10 wird in Betrieb mit Gas, zum Beispiel Argon versorgt, das durch die zueinander parallelen Lumen 22, 23, 24 fließt und aus den Gasausströmöffnungen 25 bis 27 austritt. Es umspült die Elektrode 30 bzw. deren aktives Ende 29, das den Gasstrom ionisiert und somit einen distal aus dem Instrument 10 austretenden Plasmastrom erzeugt, der auf umgebendes Gewebe trifft. Dieses ist mittels der oben erwähnten Neutralelektrode mit dem Gegenpol des Generators 13 verbunden, so dass ein Stromfluss zwischen dem aktiven Ende 29 der Elektrode 30 und dem Gewebe zustande kommt.

### Durch die Kombination aus mehreren Maßnahmen, nämlich

- gleichmäßiger Gasfluss aus den Ausströmöffnungen 25, 26, 27
- Beschichtung der Elektrode 30 z.B. mit Silber wenigstens am distalen Ende
- Konzentration der elektrischen Isolation in der Mitte des Sondenquerschnitts
kann das Instrument 10 weitgehend miniaturisiert werden. Es ist möglich, den Außendurchmesser des Sondenschlauchs 14 auf weniger als 1 mm zu reduzieren, ohne dass die von dem aktiven Ende 29 der Elektrode 30 ausgehende Wärme zu einer schnellen Schädigung des Sondenschlauchs 14 führen würde. Dies gilt sogar dann, wenn die draht- oder stabförmige, vorzugsweise gerade ausgebildete Elektrode 30 am Umfang vollflächig mit dem Kunststoff der Sonde in Kontakt steht. Eine schnelle thermische Schädigung des Sondenschlauchs wird insbesondere vermieden, wenn das aktive Ende 29, mit einer geeigneten Beschichtung, wie beispielsweise der genannten Silberbeschichtung, versehen ist, die zu einer Konzentration der elektrischen Entladung auf das distale Ende des aktiven Endes 29 der Elektrode 30 führt. Im Ergebnis wird eine hochminiaturisierbare sehr flexible Sonde erhalten, die bislang für die Argonplasmakoargulation unerreichbarer Anwendungsgebiete erschließt.

Die insbesondere am distalen Ende 16 ausgebildete Struktur des Instruments 10 kann in einem Fertigungsverfahren erzeugt werden, bei dem ein auf einen Leiter 31 extrudierter Sondenschlauch zunächst abgelängt wird, wonach an dem distalen Ende 16 eine dort vorgesehene aus Figur 4 ersichtliche Plasmakammer 33 eingearbeitet wird. Dazu werden distale Abschnitte der Trennwände 18, 19, 20 und gegebenenfalls ein Teil des Mittelabschnitts 21 zum Beispiel mechanisch entfernt. Die Elektrode 30 kann ebenfalls etwas gekürzt werden, so dass sie nicht über die Stirnseite 28 des Sondenschlauchs 14 vorsteht. Es ist aber auch möglich, die Plasmakammer 33 zu erzeugen, in dem der abgelängte Sondenschlauch 14 bei Ersteinsatz am Patienten oder auch beim Hersteller unter kontrollierten Bedingungen kurz derart in Betrieb genommen wird, dass der aktive Abschnitt 29 der Elektrode 30 in Folge der Wärmeentwicklung einen Teil der Trennwände 18, 19, 20, sowie des Mittelabschnitts 21 wegschmilzt oder -brennt. Dieser Vorgang kann unterstützt werden, indem dabei anstelle von Argon ein anderes geeignetes beispielsweise ein reaktives Gas, wie zum Beispiel CO₂, Luft oder dergleichen verwendet wird.

An der insoweit beschriebenen Sonde sind zahlreiche Abwandlungen möglich. Zum Beispiel können die Wände 18, 19, 20 wie dargestellt tangential an das Mittelstück 21 anschließen. Sie können dort aber auch radial anschließen und dann in Schrägstellung übergehen. Auch können die Wände 18, 19, 20 tangential an den Mantel 17 anschließen. Sie können dort aber auch radial anschließen und ansonsten in Schrägstellung stehen.

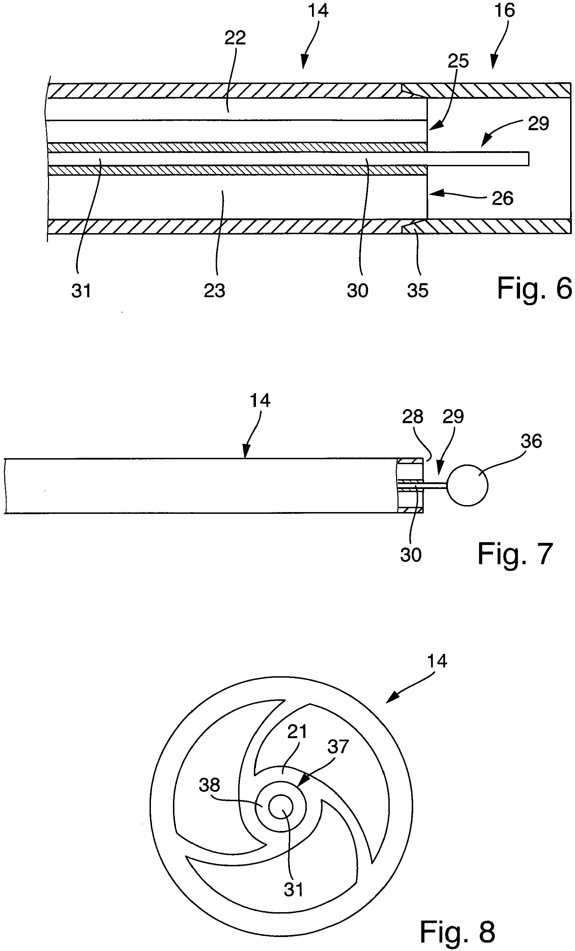

Bei allen Ausführungsformen kann das distale Ende 16 des Sondenschlauchs 14 mit einem hülsenförmigen Element 35 versehen sein, das aus einem Material gebildet ist, das sich von dem Material des Sondenschlauchs 14 unterscheidet. Figur 6 veranschaulicht dazu exemplarisch einen Sondenschlauch 14, bei dem das Element 35 durch eine Keramikhülse gebildet ist. Diese kann im stumpfen Stoß, im gestuften Stoß oder auch an einer konischen Schnittstelle an den Sondenschlauch 14 angeschlossen sein. Die Verbindung kann durch Kleben, Schweißen, z.B. Ultraschallschweißen, oder sonstige form- und/oder stoffschlüssige Verbindungstechniken erfolgen. Hinsichtlich der Ausbildung und der Positionierung der Elektrode 30 und ihres aktiven Endes 29 gelten die obigen Ausführungen zu den vorbeschriebenen Ausführungsformen entsprechend.

Bei allen vorgeschriebenen Ausführungsformen kann allerdings das aktive Ende 29 der Elektrode 30 auch, wie aus Figur 7 hervorgeht, über das stirnseitige Ende 28 des Sondenschlauchs 14 hinausragen. In diesem Fall, kann das Ende der Elektrode 30 mit einem Schutzkörper 36 beispielsweise in Form eines Isolators, Z.B. in Gestalt eines Keramikelements versehen sein. Der Schutzkörper 36 ist vorzugsweise bezüglich des aktiven Endes 29 der Elektrode 30 rotationssymmetrisch ausgebildet. Beispielsweise ist er tellerförmig, pyramidenförmig, kugelförmig, pilzförmig oder dergleichen. Vorzugsweise ist er so ausgebildet, dass von der Elektrode 30 aus gesehen alle Radialrichtungen frei sind. Damit kann sich der Plasmastrom 360° in jede beliebige Radialrichtung ausrichten. Es ist aber auch möglich, den Schutzkörper 36 asymmetrisch auszubilden und diesen mit Element 35 zu kombinieren oder auch zu verbinden. Auf diese Weise können asymmetrisch arbeitende Sonden gestaltet werden.

Die vorstehende Beschreibung der Ausführungsformen nach Figur 1 bis Figur 7 geht davon aus, dass der Leiter 31 in unmittelbaren Kontakt mit dem Material des Sondenschlauchs 14 steht. Es ist aber bei allen vorbeschriebenen Ausführungsformen auch möglich, anstelle eines blanken Leiters 31 ein Kabel 37 vorzusehen, das aus dem Leiter 31 und einer darauf aufgebrachten Kabelisolation 38 besteht. Die Kabelisolation kann z.B. durch einen isolierenden Lack oder durch einen Kunststoffschlauch gebildet sein. Auf die Kabelisolation 38 ist das Material des Sondenschlauchs 14 aufgebracht, so dass der Mittelabschnitt 21 innen aus dem Material der Kabelisolation 38 und dem Material des Sondenschlauchs besteht, das auf die Kabelisolation 38 aufgebracht ist. Mit diesem Konzept kann die Sicherheit gegen Spannungsdurchschlag weiter erhöht werden. Das Material der Kabelisolation 38 kann auf maximale Spannungsdurchschlagfestigkeit hin optimiert werden. Die Steifigkeit des Materials spielt dabei eine untergeordnete Rolle. Das Material des Sondenschlauchs 14 kann dann hingegen auf die gewünschte Flexibilität hin optimiert werden.

Es ist möglich zur Verbesserung der Spannungsdurchschlagfestigkeit an der Grenzfläche zwischen der Kabelisolation 38 und dem darauf aufgebrachten Material des Sondenschlauchs 14 eine Metallisierung vorzusehen, die eine zylinderförmige Äquipotenzialfläche festlegt. Dies kann die Spannungsdurchschlagfestigkeit erhöhen.

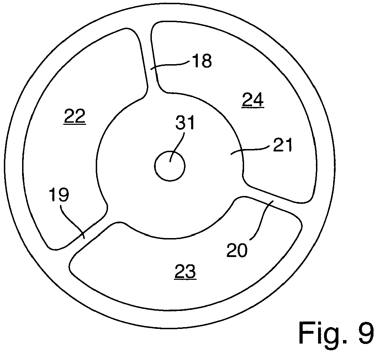

Weiter ist es möglich, die Trennwände 18, 19, 20 wie Figur 9 veranschaulicht radial auszurichten und dabei gerade oder auch gekrümmt auszubilden.

Ein erfindungsgemäßes Instrument 10 weist einen Sondenschlauch 14 auf, in dessen Zentrum ein Leiter 31 zur elektrischen Versorgung einer Elektrode 30 vorgesehen ist. Konzentrisch um den Leiter 31 herum, sind mehrere gasführende Lumen 22, 23, 24 angeordnet, die durch Trennwände 18, 19, 20 voneinander isoliert sind. Die Trennwände 18, 19, 20 halten einen zentralen den Leiter 31 beherbergenden Mittelabschnitt 21 der maßgeblich zur elektrischen Isolation des Leiters 31 dient. Mit diesem Sondendesign lassen sich besonders flexible und besonders schlanke Sonden schaffen, die eine besonders hohe Spannungsdurchschlagfestigkeit haben.

### Bezugszeichen:

- 10: Instrument
- 11: Gerät
- 12: Gasquelle
- 13: Generator
- 14: Sondenschlauch
- 15: proximales Ende des Sondenschlauchchs 14
- 16: disteles Ende des Sondenschlauchchs 14
- 17: Mantel
- 18 - 20: Trennwände
- 21: Mittelabschnitt
- 22 - 24: Lumen
- 25 - 27: Gasauströmöffnungen
- 28: stirnseitiges Ende des Sondenschlauchs 14
- 29: aktive Ende der Elektrode 30
- 30: Elektrode
- 31: Zuleitung
- 32: Kreis zur Veranschaulichung einer verbesserten elektrischen Isolierung des Leiters 31
- 33: Plasmakammer
- 34: Hülse
- 35: Element
- 36: Isolatorkörper
- 37: Kabel
- 38: Kabelisolation
- 39: radial innerer Anfang der Stirnfläche 28
- 40: Übergang zwischen Endfläche 28 und Außenfläche

## Patentansprüche

1. Instrument (10), insbesondere monopolares Instrument, insbesondere zur Argon-Plasma Koagulation von biologischem Gewebe,
mit einem Sondenschlauch (14), der mindestens zwei an eine Gasversorgungseinrichtung (12) anschließbare Lumen (22, 23) aufweist,
mit einer Elektrode (30), die in dem Sondenschlauch (14) gehalten ist und ein aktives Ende (29) aufweist,
wobei jedes Lumen (22, 23) jeweils eine Gasausströmöffnung (25, 26) aufweist, wobei mehrere Gasaustrittsöffnugen (25, 26) bei dem aktive Ende (29) der Elektrode (30) angeordnet sind.

2. Instrument nach Anspruch 1, **dadurch gekennzeichnet, dass** die Gasaustrittsöffnungen (25, 26) konzentrisch um die Elektrode (30) herum angeordnet sind.

3. Instrument nach einem der vorstehenden Ansprüche, **dadurch gekennzeichnet, dass** zwischen den Lumen (25, 26) Trennwände (18, 19) angeordnet sind, die innen an einen Mittelabschnitt (21) und außen an einen Mantel (17) anschließen, und dass die Elektrode (30) in einem Mittelabschnitt (21) isoliert angeordnet ist, wobei der Mittelabschnitt (21) eine größere radiale Dicke aufweist, als der Mantel (17).

4. Instrument nach Anspruch 3, **dadurch gekennzeichnet, dass** die Trennwände (18, 19) zur Radialrichtung geneigt angeordnet sind.

5. Instrument nach Anspruch 3 oder 4, **dadurch gekennzeichnet, dass** die Trennwände (18, 19) gekrümmt ausgebildet sind.

6. Instrument nach einem der vorstehenden Ansprüche, **dadurch gekennzeichnet, dass** der Sondenschlauch (14) außen einen Kreisquerschnitt aufweist.

7. Instrument nach einem der vorstehenden Ansprüche, **dadurch gekennzeichnet, dass** in dem Sondenschlauch (14) nur eine einzige Elektrode (30) angeordnet ist und dass die Elektrode (39) in dem Sondenschlauch (14) mittig angeordnet ist.

8. Instrument nach einem der vorstehenden Ansprüche, **dadurch gekennzeichnet, dass** die Elektrode (30) in einen Mittelabschnitt (21) des Sondenschlauchs (14) elektrisch isoliert eingebettet ist.

9. Instrument nach einem der vorstehenden Ansprüche, **dadurch gekennzeichnet, dass** der Sondenschlauch (14) einen sich über die Gasaustrittsöffnungen (25, 26) in distaler Richtung hinaus erstreckenden Mantelabschnitt (17, 35) aufweist.

10. Instrument nach Anspruch 9, **dadurch gekennzeichnet, dass** der Mantelabschnitt (35) aus einem anderen Material besteht, als der Sondenschlauch (14).

11. Instrument nach einem der vorstehenden Ansprüche, **dadurch gekennzeichnet, dass** die Elektrode (30) ein distales Ende aufweist, das innerhalb des Sondenschlauchs (14) angeordnet ist.

12. Instrument nach einem der vorstehenden Ansprüche 1 bis 10, **dadurch gekennzeichnet, dass** die Elektrode (30) eine distales Ende aufweist, das außerhalb des Sondenschlauchs (14) angeordnet ist und das mit einem Isolator (36) versehen ist.

13. Instrument nach einem der vorstehenden Ansprüche, **dadurch gekennzeichnet, dass** die Elektrode (30) durch einen freigelegten Endabschnitt eines ansonsten entlang seiner gesamten Länge in den Sondenschlauch (14) eigebetteten Drahts gebildet ist.

14. Instrument nach Anspruch 13, **dadurch gekennzeichnet, dass** der freigelegte Endabschnitt einen elektrisch leitfähigen Elektrodenaufsatz (34) trägt.

15. Instrument nach einem der vorstehenden Ansprüche, **dadurch gekennzeichnet, dass** die Elektrode (30) wenigstens abschnittsweise mit einer elektrisch leitfähigen Beschichtung versehen ist.
